# EUROPEAN PATENT APPLICATION

(11) **EP 1 769 800 A2**
(43) Date of publication of application: **04.04.2007**
(21) Application number: 07000058.3
(22) Date of filing: 15.03.2002
(51) Int. Cl.: A61K 31/565, A61K 31/566, A61K 31/567, A61P 15/12, A61P 5/30, A61P 19/10, A61P 15/02, A61P 13/02, A61P 3/06, A61P 9/00, A61P 9/10, A61P 9/14, A61P 39/06, A61P 25/28

(54) **Estrogen replacement therapy**

(30) Priority: 16.03.2001 US 276704 P
(62) Divisional of application: 02757788.1
(71) Applicant: Wyeth, Madison, NJ 07940 (US)
(72) Inventor: Pickar, James Harrison, Springfield, PA 19064 (US)
(74) Representative: Mannion, Sally Kim

(57) **Abstract**

This invention relates to pharmaceutical compositions and their use for providing estrogen replacement therapy in perimenopausal, menopausal, and Postmenopausal women through the continuous administration of conjugated estrogens, in an amount from 0.25 mg to 0.1 mg per day.

## Description

### BACKGROUND

This invention relates to methods and pharmaceutical compositions for providing estrogen replacement therapy in perimenopausal, menopausal, and postmenopausal women through the continuous administration of conjugated estrogens.

Menopause is generally defined as the last natural menstrual period and is characterized by the cessation of ovarian function, leading to the substantial diminution of circulating estrogen in the bloodstream. Menopause is usually identified, in retrospect, after 12 months of amenorrhea. It is usually not a sudden event, but is often preceded by a time of irregular menstrual cycles prior to eventual cessation of menses. Following the cessation of menstruation, the decline in endogenous estrogen concentrations is typically rapid. There is a decrease in serum estrogens from circulating levels ranging from 40-250 pg/mL of estradiol and 40-170 pg/mL of estrone during ovulatory cycles to less than 15 pg/mL of estradiol and 30 pg/mL of estrone in postmenopausal women.

As these estrogens decline during the time preceding (perimenopause) and following the menopause (postmenopause), various physiological changes may result, including vulvar and vaginal atrophy causing vaginal dryness, pruritus and dyspareunia, and vasomotor instability manifested as hot flushes. Other menopausal disturbances may include depression, insomnia, and nervousness. The long-term physiologic effects of postmenopausal estrogen deprivation may result in significant morbidity and mortality due to increase in the risk factors for cardiovascular disease and osteoporosis. Menopausal changes in blood lipid levels, a major component of the pathogenesis of coronary heart disease (CHD), may be precursors to increased incidence of ischemic heart disease, atherosclerosis, and other cardiovascular disease. A rapid decrease in bone mass of both cortical (spine) and trabecular (hip) bone can be seen immediately after the menopause, with a total bone mass loss of 1% to 5% per year, continuing for 10 to 15 years.

Estrogen replacement therapy (ERT) is beneficial for symptomatic relief of hot flushes and genital atrophy and for prevention of postmenopausal osteoporosis. ERT has been recognized as an advantageous treatment for relief of vasomotor symptoms. There is no acceptable alternative to estrogen treatment for the atrophic changes in the vagina; estrogen therapy increases the vaginal mucosa and decreases vaginal dryness. Long term ERT is the key to preventing osteoporosis because it decreases bone loss, reduces spine and hip fracture, and prevents loss of height. In addition, ERT has been shown to be effective In increasing high density lipoprotein-cholesterol (HDL-C) and in reducing low density lipoprotein cholesterol (LDL-C), affording possible protection against CHD. ERT also can provide antioxidant protection against free radical mediated disorders or disease states. Estrogens have also been reported to confer neuroprotection, and inhibit neurodegenerative disorders, such as Alzheimer's disease (see U.S. Patent 5,554,601, which is hereby incorporated by reference). The following table contains a list of some of the estrogen preparations currently available in the US and Europe. Listings of such preparations are available in such as the Physicians' Desk Reference, The Orange Book, and the European equivalents thereof.

| Estrogen replacement therapies available in the United States and/or Europe | | |
|---|---|---|
| Generic Name | Brand Name | Strength |
| **Oral estrogens** | | |
| Conjugated equine estrogens (natural) | Premarin | 0.3, 0.625, 0.9, 1.25, 2.5 mg |
| | | |
| Conjugated estrogens (synthetic) | Cenestin | 0,625, 0.9 mg |
| | | |
| Esterified estrogens (75-80% estrone sulfate, 6-15% equilin sulfate derived from plant sterols) | Estratab | 0.3, 0.625, 1.25, 2.5 mg |
| | | |
| Estropipate (Piperazine estrone sulfate) | Ogen Ortho-Est | 0.625, 1,25, 2.5 mg |
| | | |
| Micronized estradiol | Estrace | 0.5, 1.0, 2.0 mg |
| | | |
| Raloxifene (SERM) | Evista | 60 mg |
| | | |
| Esterified estrogens and methylestosterone | Estratest | 1.25 mg esterified estrogen and |
| | | 2.5 mg methylestosterone |
| | Estratest HS | 0,625 mg esterified estrogen and |
| | | 1.25 mg methylestosterone |
| | | |
| Estradiol valerate | Climaval | 1 mg, 2 mg |
| Estradiol | Elleste Solo | 1 mg, 2 mg |
| Estradiol | Estrofem | 2 mg |
| Estradiol | Estrofem Forte | 4 mg |
| Piperazine esterone sulfate | Harmogen | 1.5 mg |
| | | |
| Combination Estrone | Hormonin | 1.4 mg |
| Product: Estradiol | | 0.6 mg |
| Estriol | | 0.27 mg |
| | | |
| Estradiol valerate | Progynova | 1 mg, 2 mg |
| Estradiol | Zumenon | 1 mg, 2 mg |
| | | |

| **Transdermal estrogens** | | |
|---|---|---|
| Estradiol | Alora (twice wkly) | 0.025, 0.0375, 0.05, 0.075, |
| | Climara (weekly) | 0.1 mg of estradiol released daily (dose options for various products) |
| | Estraderm (2x wkly) | |
| | Fem Patch (wkly) | |
| | Vivelle (twice wkly) | |
| Estradiol | Dermestril | 25, 50, 100 µg |
| Estradiol | Estraderm | 25, 50, 100 µg |
| Estradiol | Evorel (Systen) | 25, 50, 75, 100 µg |
| Estradiol | Fematrix | 40, 80 µg |
| Estradiol | Menorest | 25, 37.5, 50, 75 µg |
| Estradiol | Progynova TS And TS Forte (Climara) | 50, 100 µg |
| | | |

| **Vaginal estrogens** | | |
|---|---|---|
| Conjugated equine estrogens | Premarin vaginal cream | 0.625 mg/g |
| Dienestrol | Ortho dienestrol cream | 0.1 mg/g |
| Estradiol | Estring | 7.5 µg |
| Estroplpate | Ogen vaginal cream | 1.5 mg/g |
| Micronized estradiol | Estrace vaginal cream | 1.0 mg/g |

To minimize the occurrence of estrogen-related side effects and to maximize the benefit-risk ratio, the lowest dose effective in relief of symptoms and prevention of osteoporosis should be used. Although ERT reduces the relative risk (RR) for ischemic heart disease (RR, 0.50) and osteoporosis (RR, 0.40), the relative risk of endometrial cancer for postmenopausal women with a uterus may be increased. There are extensive clinical data showing that the relative risk of endometrial cancer can be reduced by the addition of a progestin, either sequentially or continuously. The addition of a progestin to estrogen therapy prevents estrogen-induced endometrial proliferation.

The addition of a progestin to ERT regimens, however, may ameliorate some of the favorable estrogen effects on lipids and may potentially impair glucose tolerance, it has desirably been an objective of HRT regimens to use the lowest dosage of progestin that will minimize or eliminate endometrial hyperplasia. It is therefore an object of this invention to provide low dosage ERT regimens that may minimize endometrial proliferation so that the need for concomitant progestin administration is diminished. Accordingly, the ERT regimens covered by this invention are particularly useful in treating perimenopausal, menopausal, or postmenopausal women when accompanied by adequate physician monitoring, and are also particularly useful in treating subgroups of hysterectomized or progestin intolerant women,

### DESCRIPTION OF THE INVENTION

The purpose of this invention is to provide the significant benefits of a commercially successful ERT product, such as PREMARIN (0.3 mg, 0.625 mg, 0.9 mg, 1.25 mg, or 2.5 mg conjugated equine estrogens, USP), while lowering the dosage of conjugated estrogens below that which has previously been demonstrated to be effective. This invention provides a method of treating or inhibiting menopausal or postmenopausal disorders in a perimenopausal, menopausal, or postmenopausal woman in need thereof, which comprises providing to said woman, continuously and uninterruptedly over the treatment period, a daily dosage in an amount from about 0.25 mg to about 0.1 mg conjugated estrogens (natural or synthetic). The dosage is preferably provided as a pharmaceutical composition for use in treating menopausal or postmenopausal disorders. This invention further provides a pharmaceutical pack containing the daily dosage units of conjugated estrogen.

Conjugated estrogens refer to estrogenic steroidal substances in which one or more functional groups (typically hydroxyl groups) on the steroid exists as a conjugate (typically a sulfate or glucumnide). The conjugated estrogens may be a single conjugated estrogen, or may consist of mixtures of various conjugated estrogens. Numerous conjugated estrogens are described in the literature or are commercially available that are capable of being formulated for use in this invention either as a unitary estrogen, or may be mixed together with other synthetic and/or natural estrogens.

Conjugated estrogens may also contain other steroidal or non-steroidal compounds, which may, or may not, contribute to the overall biological effect. Such compounds include, but are not limited to, unconjugated estrogens, androstanes, and pregnanes. Preferred conjugated estrogens for use in this invention are PREMARIN (conjugated equine estrogens, USP, conforming with the monograph for conjugated estrogens in USP25) and CENESTIN (synthetic conjugated estrogens, A).

PREMARIN (conjugated estrogens tablets, USP) for oral administration contains a mixture of estrogens obtained exclusively from natural sources, occurring as the sodium salts of water-soluble estrogen sulfates blended to represent the average composition of material derived from pregnant mares' urine. It is a mixture of sodium estrone sulfate and sodium equilin sulfate, and at least the following 8 concomitant components, also as sodium sulfate conjugates: 17α-dihydmequilin, 17α-estradiol, Δ8,9-dehydroestrone, 17β-dihydroequilin, 17β-estradiol, equilenin, 17α-dihydroequilenin, and 17β-dihydroequilenin. PREMARIN is indicated in the treatment of moderate to severe vasomotor symptoms associated with the menopause; treatment of vulvar and vagina! atrophy; and prevention of osteoporosis, as well as other indications approved for estrogen products.

CENESTIN (synthetic conjugated estrogens, A) tablets for oral administration contain a blend of 9 synthetic estrogenic substances: sodium estrone sulfate, sodium 17α-dihydroequilin sulfate, sodium 17α-estradiol sulfate, sodium equilenin sulfate, sodium 17α-dihydroequilenin sulfate, sodium equilin sulfate, sodium 17β-dihydro-equilin sulfate, sodium 17β-estradiol sulfate, sodium 17α-dihydroequilenin sulfate. CENESTIN is indicated in the treatment of moderate to severe vasomotor symptoms associated with the menopause.

PREMARIN and CENESTIN are available from commercial sources (Wyeth-Ayerst - PREMARIN; Duramed - CENESTIN).

It is preferred that the conjugated estrogen constituent is PREMARIN. It is preferred that the dosage of PREMARIN is from about 0.25 mg per day to about 0.1 mg per day, and is more preferred that the dosage of PREMARIN is from about 0.2 mg per day to about 0.1 mg per day, with a daily dosage of about 0.2 mg being specifically preferred. It is also preferred that the ERT regimens described herein be administered to hysterectomized women, or women with an uterus accompanied by careful physician monitoring for endometrial hyperplasia.

If desired, the conjugated estrogen regimens of this invention can be administered in conjunction with a progestin, particularly medroxyprogesterone acetate (MPA, commercially available from Wyeth-Ayerst). When MPA is used as the progestin, it is preferred that the daily dosage of MPA is 2.5 mg or less. Such concomitant administration can be as a combination (as defined below), or that the progestin can be provided for only part of the treatment period. For example, PREMARIN may administered for 28-days per 28-day treatment period, and MPA may be administered on days 15-28 of the same 28-day treatment period.

As used in accordance with this invention, the term "menopausal or postmenopausal disorder" refers to conditions, disorders, or disease states that are at least partially caused by the decreased estrogen production occurring during the perimenopausal, menopausal, or post-menopausal stages of a woman's life. Such disorders typically include, but are not limited to, one or more of, vaginal and vulvar atrophy, vasomotor instability, urinary incontinence, and increased risk of developing osteoporosis, cardiovascular disease, and diseases related to the oxidative damage from free radicals. As used herein, menopausal also includes conditions of decreased estrogen production that may be surgically, chemically, or be caused by a disease state which leads to premature diminution or cessation of ovarian function.

The term "daily" means that the dosage is to be administered at least once daily. The frequency is preferred to be once daily, but may be more than once daily, provided that any specified daily dosage is not exceeded,

The term "continuous and uninterrupted" means that there is no break in the treatment regimen, during the treatment period. Thus, "continuous, uninterrupted administration" of a combination, means that the combination is administered at least once daily during the entire treatment period. It is expected that the treatment period for the ERT regimens of this invention will be for at least 30 days, preferably 120 days, and most preferably as long term treatment, and possibly indefinite, as one of the primary reasons for administering ERT is to treat or inhibit menopausal or postmenopausal disorders. Treatment periods also may vary depending on the symptoms to be treated. For example, for the treatment of vasomotor symptoms, it is preferred that the treatment may last from one month to several years, depending on the severity and duration of the symptoms. Physician evaluation along with patient interaction will assist the determination of the duration of treatment. For the treatment or inhibition of osteoporosis, it is preferred that the treatment period could last from six months to a number of years, or indefinitely.

This invention, also covers short term treatments or treatments of a finite term, that may be less than the 30 day preferred treatment period. It is anticipated that a patient may miss, or forget to take, one or a few dosages during the course of a treatment regimen, however, such patient is still considered to be receiving continuous, uninterrupted administration.

The term "fixed daily dosage" means that the same dosage is given every day during the treatment period. One aspect of this invention also covers situations in which a fixed daily dosage of the ERT regimen is not given every day during the treatment period. For example, the dosage of a patient may need to be adjusted (either up or down), to achieve the desired effect during the middle of a treatment period.

The term "providing," with respect to providing a dosage of one or both of the components of this invention, means either directly administering such a component of this invention, or administering a prodrug, derivative, or analog which will form the equivalent amount of the component within the body.

It is preferred that the conjugated estrogens of this invention are provided orally. The specific dosages of conjugated estrogens plus MPA combinations of this invention that are disclosed herein are oral dosages.

The term "combination" means that the daily dosage of each of the components of the combination is administered during the treatment day. The components of the combination are preferably administered at the same time; either as a unitary dosage form containing both components, or as separate dosage units; the components of the combination can be administered at different times during the day, provided that the desired daily dosage is achieved.

In accordance with this invention, continuously and uninterruptedly providing a daily dosage from about 0.25 mg to about 0.1 mg conjugated estrogens is useful in treating or inhibiting menopausal or postmenopausal disorders in perimenopausal, menopausal, or postmenopausal women. More particularly, the combinations described herein are useful in treating or inhibiting vaginal or vulvar atrophy; atrophic vaginitis; vaginal dryness; pruritus; dyspareunia; dysuria; frequent urination; urinary incontinence; urinary tract Infections; vasomotor symptoms, including hot flushes, myalgia, arthralgia, insomnia, irritability, and the like; inhibiting or retarding bone demineralization; increasing bone mineral density; and treating or inhibiting osteoporosis.

The combinations of this invention also exert a cardioprotective effect in perimenopausal, menopausal, and postmenopausal women, and are therefore useful in lowering chotesterol, Lp(a), and LDL levels; inhibiting or treating hypercholesteremia; hyperlipidemia; cardiovascular disease; atherosclerosis; peripheral vascular disease; restenosis, and vasospasm; and inhibiting vascular wall damage from cellular events leading toward immune mediated vascular damage.

The combinations of this invention are antioxidants, and are therefore useful in inhibiting disorders or disease states which involve free radicals. More particularly, the combinations of this invention are useful in treating or inhibiting free radical involvement in the development of cancers, central nervous system disorders, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, amyotrophic lateral sclerosis, aging effects, adult respiratory distress syndrome, central nervous system trauma and stroke, or injury during reperfusion procedures.

The combinations of this invention are useful in treating or inhibiting dementias, neurodegenerative disorders, and Alzheimer's disease; providing neuroprotection or cognition enhancement.

Conjugated estrogens may be formulated neat or may be combined with one or more pharmaceutically acceptable carriers for administration. For example, solid carriers include starch, lactose, dicalcium phosphate, microcrystalline cellulose, sucrose and kaolin, while liquid carriers include sterile water, polyethylene glycols, non-ionic surfactants and edible oils such as corn, peanut and sesame oils, as are appropriate to the nature of the active ingredient and the particular form of administration desired. Adjuvants customarily employed in the preparation of pharmaceutical compositions may be advantageously included, such as flavoring agents, coloring agents, preserving agents, and antioxidants, for example, vitamin E, ascorbic acid, BHT and BHA.

The preferred pharmaceutical compositions from the standpoint of ease of preparation and administration are solid compositions, particularly tablets and hard-filled or liquid-filled capsules. Oral administration of the compounds is preferred.

In the Physicians' Desk Reference, PREMARIN is described as containing calcium phosphate tribasic, calcium sulfate, camuaba wax, cellulose, glyceryl momooleate, lactose, magneseum stearate, methyl cellulose, pharmaceutical glaze, polyethylene glycol, stearic acid, sucrose, and titanium dioxide as inactive ingredients. This would be a typical formulation for PREMARIN.

CENESTIN is described as containing ethylcellulose, hydroxypropyl methylcellulose, lactose monohydrate, magnesium stearate, polyethylene glycol, polysorbate 80, pregelatinized starch, titanium dioxide, and triethyl citrate as inactive ingredients. Formulations covering CENESTIN are described In US Patent 5,908,638, which is hereby incorporated by reference. This would be a typical formulation for CENESTIN.

Conjugated estrogens may be formulated in a core containing the conjugated estrogens, and several components including alcohol, hydroxypropyl methyl cellulose, lactose monohydrate, magnesium stearate, and starch. The core can be covered with a coating made from components such as ethylcellulose, and methyl citrate. Conjugated estrogens can be incorporated in granules, spheroids or other multiparticulate forms, and, if necessary, coated to provide adequate stability.

This invention also provides a pharmaceutical pack, containing any number of daily pharmaceutical dosage units. Preferably, and conventionally, the pack contains 28 tablets or multiples thereof. The pack should indicate that the dosage units are to be taken consecutively on a daily basis until the treatment period has ended, or until the pack has been completed. The next pack should be started on the next consecutive day.

The ERT regimens described in this invention may also be administered as a transdermal patch or as a vaginal cream. For example, PREMARIN vaginal cream containing 0.625 mg conjugated equine estrogens, USP, is formulated to contain USP in a nonliquefying base containing cetyl esters wax, cetyl alcohol, white wax, glyceryl monostearate, propylene glycol monostearate, methyl stearate, benzyl alcohol, sodium lauryl sulfate, glycerin, and mineral oil as excipients. ERT regimens covered by this invention can be formulated similarly.

For the purposes of this disclosure, transdermal administrations are understood to include all administrations across the surface of the body and the inner linings of bodily passages including epithelial and mucosal tissues. Such administrations may be carried out using the present compounds, or pharmaceutically acceptable salts thereof, in lotions, creams, foams, patches, suspensions, solutions, and suppositories (rectal and vaginal).

Transdermal administration may be accomplished through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active Ingredient may also be suitable. A variety of occlusive devices may be used to release the active ingredient into the blood stream such as a semi-permeable membrane covering a reservoir containing the active ingredient with or without a carrier, or a matrix containing the active ingredient. Other occlusive devices are known in the literature.

## Claims

1. A method of treating or inhibiting menopausal or postmenopausal disorders in a perimenopausal, menopausal, or postmenopausal woman in need thereof, which comprises orally providing to said woman continuously and uninterruptedly over the treatment period, a daily dosage in an amount from about 0.25 mg to about 0.1 mg conjugated estrogens.

2. The method according to claim 1, wherein the conjugated estrogens is conjugated equine estrogens, USP.

3. The method according to claim 2, wherein the daily dosage of conjugated equine estrogens is from about 0.2 mg to about 0.9 mg.

4. The method according to claim 3, wherein the daily dosage of conjugated equine estrogens, USP is about 0.2 mg.

5. The method according to claim 1, wherein the conjugated estrogens is synthetic conjugated estrogens, A.

6. A method of treating or inhibiting vasomotor symptoms in a perimenopausal, menopausal, or postmenopausal woman in need thereof, which comprises orally providing to said woman continuously and uninterruptedly over the treatment period, a daily dosage in an amount from about 0.25 mg to about 0.1 mg conjugated estrogens.

7. The method according to claim 6, wherein the conjugated estrogens is conjugated equine estrogens, USP.

8. The method according to claim 7, wherein the daily dosage of conjugated equine estrogens is from about 0.2 mg to about 0.1 mg.

9. The method according to claim 8, wherein the daily dosage of conjugated equine estrogens, USP is about 0.2 mg.

10. The method according to claim 6, wherein the vasomotor symptom is hot flushes.

11. The method according to claim 6, wherein the conjugated estrogens is synthetic conjugated estrogens, A.

12. A method of inhibiting or retarding bone demineralization or treating or inhibiting osteoporosis in a perimenopausal, menopausal, or postmenopausal woman in need thereof, which comprises orally providing to said woman continuously and uninterruptedly over the treatment period, a daily dosage in an amount from about 0.25 mg to about 0.1 mg conjugated estrogens.

13. The method according to claim 12, wherein the conjugated estrogens is conjugated equine estrogens, USP.

14. The method according to claim 13, wherein the daily dosage of conjugated equine estrogens is from about 0.2 mg to about 0.1 mg.

15. The method according to claim 14, wherein the daily dosage of conjugated equine estrogens, USP is about 0.2 mg.

16. A method of treating or inhibiting vaginal or vulvar atrophy, atrophic vaginitis; vaginal dryness; pruritus; dyspareunia; dysuria; frequent urination; urinary incontinence; urinary tract infections in a perimenopausal, menopausal, or postmenopausal woman in need thereof, which comprises orally providing to said woman continuously and uninterruptedly over the treatment period, a daily dosage in an amount from about 0.25 mg to about 0.1 mg conjugated estrogens.

17. The method according to claim 16, wherein the conjugated estrogens is conjugated equine estrogens, USP.

18. The method according to claim 17, wherein the daily dosage of conjugated equine estrogens is from about 0.2 mg to about 0.1 mg.

19. The method according to claim 18, wherein the daily dosage of conjugated equine estrogens, USP is about 0.2 mg.

20. A method of lowering cholesterol, Lp(a), or LDL levels; inhibiting or treating hypercholesteremia; hyperlipidemia; cardiovascular disease; atherosclerosis; peripheral vascular disease; restenosis, vasospasm; or inhibiting vascular wall damage from cellular events leading toward immune mediated vascular damage, in a perimenopausal, menopausal, or postmenopausal woman in need thereof, which comprises orally providing to said woman continuously and uninterruptedly over the treatment period, a daily dosage in an amount from about 0.25 mg to about 0.1 mg conjugated estrogens.

21. The method according to claim 20, wherein the conjugated estrogens is conjugated equine estrogens, USP.

22. The method according to claim 21, wherein the daily dosage of conjugated equine estrogens is from about 0.2 mg to about 0.1 mg.

23. The method according to claim 22, wherein the daily dosage of conjugated equine estrogens, USP is about 0.2 mg.

24. A method of treating or inhibiting free radical involvement in the development of cancers, central nervous system disorders, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, amyotrophic lateral sclerosis, aging effects, adult respiratory distress syndrome, central nervous system trauma and stroke, or injury during reperfusion procedures in a perimenopausal, menopausal, or postmenopausal woman in need thereof, which comprises orally providing to said woman continuously and uninterruptedly over the treatment period, a daily dosage in an amount from about 0.25 mg to about 0.1 mg conjugated estrogens.

25. The method according to claim 24, wherein the conjugated estrogens is conjugated equine estrogens, USP.

26. The method according to claim 25, wherein the daily dosage of conjugated equine estrogens is from about 0.2 mg to about 0.1 mg.

27. The method according to claim 26, wherein the daily dosage of conjugated equine estrogens, USP is about 0.2 mg.

28. A method of treating or inhibiting dementias, neurodegenerative disorders, and Alzheimer's disease; providing neuroprotection or cognition enhancement in a perimenopausal, menopausal, or postmenopausal woman in need thereof, which comprises orally providing to said woman continuously and uninterruptedly over the treatment period, a daily dosage in an amount from about 0.25 mg to about 0.1 mg conjugated estrogens.

29. The method according to claim 28. wherein the conjugated estrogens is conjugated equine estrogens, USP.

30. The method according to claim 31, wherein the daily dosage of conjugated equine estrogens is from about 0.2 mg to about 0.1 mg.

31. The method according to claim 30, wherein the daily dosage of conjugated equine estrogens, USP is about 0.2 mg.

32. A pharmaceutical composition for use in treating menopausal or postmenopausal disorders, which comprises dosage in an amount from about 0.25 mg to about 0.1 mg conjugated estrogens, and a pharmaceutical carrier.

33. The composition according to claim 32, wherein the conjugated estrogens is conjugated equine estrogens, USP.

34. The composition according to daim 33, wherein the dosage of conjugated equine estrogens is from about 0.2 mg to about 0.1 mg.

35. The composition according to claim 34, wherein the dosage of conjugated equine estrogens, USP is about 0.2 mg.

36. A pharmaceutical dosage unit which comprises conjugated estrogens, a dosage in an amount from about 0.25 mg to about 0.1 mg conjugated estrogens and a pharmaceutical carrier.

37. The dosage unit according to claim 36, wherein the conjugated estrogens is conjugated equine estrogens, USP.

38. The dosage unit according to claim 37, wherein the dosage of conjugated equine estrogens is from about 0.2 mg to about 0.1 mg.

39. The dosage unit according to claim 38, wherein the dosage of conjugated equine estrogens, USP is about 0.2 mg.

40. A method of minimizing or reducing levels of breast pain in a woman receiving hormone replacement therapy, which comprises orally providing to said woman continuously and uninterruptedly over the treatment period, a daily dosage in an amount from about 0.25 mg to about 0.1 mg conjugated estrogens.

41. The method according to claim 40, wherein the conjugated estrogens is conjugated equine estrogens, USP.

42. The method according to claim 41, wherein the daily dosage of conjugated equine estrogens is from about 0.2 mg to about 0.1 mg.

43. The method according to daim 42, wherein the daily dosage of conjugated equine estrogens, USP is about 0.2 mg.

44. A method of minimizing spotting or breakthrough bleeding; or achieving amenorrhea in a woman receiving hormone replacement therapy, which comprises orally providing to said woman continuously and uninterruptedly over the treatment period, a daily dosage in an amount from about 0.25 mg to about 0.1 mg.

45. The method according to claim 44, wherein the conjugated estrogens is conjugated equine estrogens, USP.

46. The method according to claim 45, wherein the daily dosage of conjugated equine estrogens is from about 0.2 mg to about 0.1 mg.

47. The method according to claim 46, wherein the daily dosage of conjugated equine estrogens, USP is about 0.2 mg.

48. A method of increasing bone mineral density in a perimenopausal, menopausal, or postmenopausal woman in need thereof, which comprises orally providing to said woman continuously and uninterruptedly over the treatment period, a daily dosage in an amount from about 0.25 mg to about 0.1 mg conjugated estrogens.

49. The method according to claim 48, wherein the conjugated estrogens is conjugated equine estrogens, USP.

50. The method according to claim 49, wherein the daily dosage of conjugated equine estrogens is from about 0.2 mg to about 0.1 mg.

51. The method according to claim 50, wherein the daily dosage of conjugated equine estrogens, USP is about 0.2 mg.

52. A pharmaceutical pack for use in the treatment of menopausal or postmenopausal disorders comprising a plurality of pharmaceutical dosage units as defined in any of claims 36 to 39 for continuous uninterrupted daily administration of a daily dosage

53. Use of conjugated estrogens in the manufacture of a pharmaceutical composition as defined in any of claims 32 to 35 or one or more pharmaceutical dosage units as defined in any of claims 36 to 39, for the treatment of menopausal or post-menopausal disorders.

54. Use of conjugated estrogens in the manufacture of a pharmaceutical pack as defined in claim 52, for the treatment of menopausal or post-menopausal disorders.

55. Use of conjugated estrogens according to claim 52 or 53 for the treatment or inhibition of vasomotor symptoms in a perimenopausal, menopausal or postmenopausal woman in need thereof.

56. Use of conjugated estrogens according to claim 55 wherein the vasomotor symptom is hot flushes.

57. Use of conjugated estrogens according to claim 52 or 53 for inhibiting or retarding bone demineralization or treating or inhibiting osteoporosis in a perimenopausal, menopausal, or postmenopausal woman in need thereof.

58. Use of conjugated estrogens according to claim 52 or 53 for treating or inhibiting vaginal or vulvar atrophy; atrophic vaginitis; vaginal dryness; pruritus; dyspareunia; dysuria; frequent urination; urinary incontinence; urinary tract infections in a perimenopausal, menopausal, or postmenopausal woman in need thereof.

59. Use of conjugated estrogens according to claim 52 or 53 for lowering cholesterol, Lp(a), or LDL levels; inhibiting or treating hypercholesteremia; hyperlipidemia; cardiovascular disease; atherosclerosis; peripheral vascular disease; restenosis, vasospasm; or inhibiting vascular wall damage from cellular events leading toward immune mediated vascular damage, in a perimenopausal, menopausal, or postmenopausal woman in need thereof

60. Use of conjugated estrogens according to claim 52 or 53 for treating or inhibiting free radical involvement in the development of cancers, central nervous system disorders, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, amyotrophic lateral sclerosis, aging effects, adult respiratory distress syndrome, central nervous system trauma and stroke, or injury during reperfusion procedures in a perimenopausal, menopausal, or postmenopausal woman in need thereof

61. Use of conjugated estrogens according to claim 52 or 53 for treating or inhibiting dementias, neurodegenerative disorders, and Alzheimer's disease; providing neuroprotection or cognition enhancement in a perimenopausal, menopausal, or postmenopausal woman in need thereof

62. Use of conjugated estrogens according to claim 52 or 53 for minimizing or reducing levels of breast pain in a woman receiving hormone replacement therapy.

63. Use of conjugated estrogens according to claim 52 or 53 for minimizing spotting or breakthrough bleeding; or achieving amenorrhea in a woman receiving hormone replacement therapy.

64. Use of conjugated estrogens according to claim 52 or 53 for increasing bone mineral density in a perimenopausal, menopausal, or postmenopausal woman in need thereof
